# DEMANDE DE BREVET EUROPEEN

(11) **EP 1 506 768 A1**
(43) Date de publication de la demande: **16.02.2005**
(21) Numéro de dépôt: 04291919.1
(22) Date de dépôt: 28.07.2004
(51) Int. Cl.: A61K 7/09

(54) **Procédé de déformation permanente des cheveux comprenant une étape de précipitation in situ d'un polymère anionique ou amphotère**

(30) Priorité: 07.08.2003 FR 0309737; 14.05.2004 FR 0405271
(71) Demandeur: L'OREAL, 75008 Paris (FR)
(72) Inventeur: Campain, Catherine, 75019 Paris (FR); Devin-Baudoin, Priscille, 92170 Vanves (FR)
(74) Mandataire: Casalonga, Axel

(57) **Abrégé**

La présente invention concerne un procédé de déformation permanente des cheveux, comprenant, dans l'ordre, les étapes suivantes :
- application, sur les zones à déformer, d'une solution d'un polymère anionique ou amphotère dans un milieu contenant de l'eau et, éventuellement, un ou plusieurs solvants cosmétiquement acceptables,
- application, sur les zones traitées dans l'étape (a), d'une composition réductrice contenant un agent réducteur capable de couper les ponts disulfure de la kératine et une quantité suffisante de cations métalliques et/ou d'ions ammonium pour faire précipiter *in situ* le polymère anionique ou amphotère,
- mise en forme des cheveux ainsi traités suivie d'un temps de pose de la composition réductrice,
- application, sur les zones traitées dans les étapes (a) et (b), d'une composition fixatrice contenant un agent oxydant, suivie d'un temps de pose de la composition oxydante, et
- rinçage des cheveux,
ainsi qu'un agent multi-composants permettant de mettre en oeuvre ce procédé.

## Description

La présente invention concerne un procédé de déformation permanente des cheveux comprenant une étape de précipitation *in situ* d'un polymère hydrosoluble, ainsi qu'un agent multi-composants pour mettre en oeuvre un tel procédé.

L'une des techniques couramment utilisées pour obtenir une déformation permanente des cheveux consiste à les traiter successivement avec un agent réducteur capable de couper les ponts disulfure de la kératine, puis avec un agent oxydant rétablissant les ponts disulfure tout en maintenant les cheveux sous une contrainte mécanique, par exemple au moyen de bigoudis.

La traction exercée par les bigoudis permet d'obtenir un décollement de racines, mais implique également l'ondulation des cheveux. Or, de nombreuses femmes souhaitent augmenter le volume de leur chevelure sans pour autant avoir des cheveux frisés.

On peut, certes, utiliser des bigoudis de grande taille pour atténuer l'aspect frisé et obtenir simplement une légère ondulation, mais la tenue de telles déformations permanentes n'est pas très bonne.

Un autre inconvénient des techniques de déformation permanente usuelles réside dans la sensibilisation des cheveux résultant du traitement répété des cheveux par des agents réducteurs et oxydants forts.

Pour remédier à cet inconvénient, il a déjà été proposé d'utiliser des compositions réductrices gélifiées permettant de localiser le produit au niveau des racines et d'éviter ainsi une dégradation chimique du cheveu sur toute sa longueur. L'utilisation d'une composition réductrice gélifiée ne dispense toutefois pas de l'étape d'enroulement des cheveux sur des bigoudis et cette technique reste par conséquent longue et difficile a mettre en oeuvre par l'utilisateur lui-même et donne un résultat de déformation peu durable.

Il existe un certain nombre de tentatives pour obtenir une déformation permanente des cheveux sans utilisation de moyens mécaniques de mise en forme tels que des bigoudis. Ainsi, la demande WO99/18922 divulgue un procédé de déformation permanente des cheveux utilisant une composition réductrice épaissie ayant un pouvoir collant modéré. Cette composition réductrice permet la mise en forme des cheveux sans application d'une contrainte de tension. La composition oxydante est appliquée directement sur les cheveux traités avec la composition réductrice, sans rinçage intermédiaire destiné à éliminer l'agent réducteur. Ce document n'envisage à aucun moment la déformation partielle des cheveux, limitée à certaines zones, par exemple à la zone proche des racines.

La demande WO01/95868 divulgue un procédé de déformation permanente des cheveux limitée à certaines zones, par exemple celle proche des racines, n'utilisant pas de moyens mécaniques de mise sous tension des cheveux tels que des bigoudis. Le maintien de la forme imposée aux cheveux pendant les phases de réduction et d'oxydation est assuré par la composition réductrice elle-même qui contient une cire, des particules hydrophiles absorbantes, en particulier des particules de farine, et un agent réducteur absorbé par les particules hydrophiles. Ce procédé est relativement complexe, car il implique la fusion de la cire dans un dispositif de chauffage approprié et le mélange de la cire fondue avec les particules hydrophiles absorbantes et l'agent réducteur. L'application du mélange chaud sur les cheveux demande au coiffeur une certaine dextérité et est difficile à réaliser par l'utilisateur lui-même. Par ailleurs, on observe un décapage de la couleur relativement important lorsque cette technique est utilisée pour la déformation permanente de cheveux colorés.

La demanderesse a mis au point un nouveau procédé de déformation permanente des cheveux, sensiblement plus facile à mettre en oeuvre que celui décrit dans demande WO01/95868, et qui ne nécessite pas une préparation préalable de la composition réductrice au moyen d'un dispositif de chauffage. Dans le procédé de la présente invention, le maintien de la forme imposée aux cheveux pendant les étapes de réduction et d'oxydation est assuré, non pas par la solidification d'une substance fluide ou malléable, mais grâce à la précipitation *in situ* d'un polymère appliqué sur les cheveux sous forme de solution.

Ce procédé s'avère d'une facilité étonnante, impliquant simplement l'application successive de trois compositions (composition de polymère, composition réductrice et composition fixatrice) et une mise en forme manuelle des cheveux ne nécessitant aucun moyen mécanique de mise sous tension.

La présente invention a par conséquent pour objet un procédé de déformation permanente des cheveux, comprenant, dans l'ordre, les étapes suivantes :
(a) application, sur les zones à déformer, d'une solution d'un polymère anionique ou amphotère dans un milieu contenant de l'eau et, éventuellement, un ou plusieurs solvants cosmétiquement acceptables,
(b) application, sur les zones traitées dans l'étape (a), d'une composition réductrice contenant un agent réducteur capable de couper les ponts disulfure de la kératine et une quantité suffisante de cations métalliques et/ou d'ions ammonium pour faire précipiter *in situ* le polymère anionique ou amphotère,
(c) mise en forme des cheveux ainsi traités suivie d'un temps de pose de la composition réductrice,
(d) application, sur les zones traitées dans les étapes (a) et (b), d'une composition fixatrice contenant un agent oxydant, suivie d'un temps de pose de la composition oxydante, et
(e) rinçage des cheveux.

L'invention a également pour objet un agent multi-composants pour la déformation permanente des cheveux comprenant,
- en tant que premier composant (composant A), une solution d'un polymère anionique ou amphotère dans un milieu contenant de l'eau et, éventuellement, un ou plusieurs solvants cosmétiquement acceptables, capable de former un précipité solide en présence d'une quantité suffisante de cations métalliques ou d'ions d'ammonium,
- en tant que deuxième composant (composant B), une composition réductrice contenant un agent réducteur capable de couper les ponts disulfure de la kératine, et une quantité suffisante de cations métalliques et/ou d'ions ammonium pour faire précipiter le polymère anionique ou amphotère du premier composant, et
- en tant que troisième composant (composant C), une composition oxydante contenant un agent oxydant capable de rétablir les ponts disulfure de la kératine.

Cet agent multi-composants se présente de préférence sous forme d'un kit multi-compartiments comportant au moins trois compartiments contenant respectivement les trois composants A, B et C ci-dessus, emballés et commercialisés dans un même emballage.

Les solvants organiques cosmétiquement acceptables que l'on peut utiliser dans la présente invention pour dissoudre le polymère anionique ou amphotère sont des liquides à température ambiante et à pression atmosphérique. Ces solvants sont de préférence des monoalcools, des polyols ou des éthers de ces alcools ou polyols. On peut citer par exemple l'éthanol, l'isopropanol, le glycérol, le propylèneglycol, l'éther monométhylique de propylèneglycol. L'éthanol est un solvant particulièrement préféré.

La solution de polymère anionique ou amphotère (composant A) peut se présenter sous une forme épaissie, voire gélifiée, permettant une application ciblée au niveau de certaines zones des cheveux.

Les polymères anioniques ou amphotères utilisés dans le composant A de la présente invention sont solubles dans le milieu liquide utilisé. Sans vouloir être tenue par une quelconque théorie, la demanderesse pense qu'après application de la solution de polymère sur les cheveux, on augmente fortement la force ionique du milieu par addition de la composition réductrice fortement chargée en cations métalliques et/ou ions ammonium (sous forme de sels). Il se produit alors une contraction de la pelote macromoléculaire aboutissant au final à la précipitation du polymère.

La force ionique nécessaire pour obtenir la précipitation *in situ* du polymère dépend d'un certain nombre de facteurs tels que le nombre de charges du polymère, le caractère plus ou moins hydrophobe du polymère sous sa forme non chargée, la masse moléculaire du polymère, la concentration du polymère dans le composant (A) ou encore le pH du milieu obtenu par mélange *in situ* des composants (A) et (B).

Cette force ionique peut être amenée par des sels minéraux ou organiques présents dans la composition réductrice B, mais également par l'agent réducteur lui même lorsqu'il est sous forme de sel.

La demanderesse a constaté qu'une concentration en cations métalliques et/ou ammonium dans la composition réductrice appliquée (composant (B)) supérieure à 0,3 M était généralement suffisante pour obtenir un dépôt de polymère précipité d'une qualité satisfaisante permettant le maintien de la forme de la chevelure pendant les étapes de réduction et d'oxydation. De préférence, cette concentration est comprise entre 0,5 et 6 M.

On peut, en principe, utiliser n'importe quel polymère anionique ou amphotère, naturel ou synthétique.

Une famille de polymères préférés utilisables pour le procédé de l'invention est formée par les copolymères synthétiques obtenus par polyaddition d'un mélange de monomères anioniques et de monomères non-ioniques, ou par polyaddition d'un mélange de monomères anioniques, non-ioniques et cationiques.

Les monomères anioniques utilisés pour la synthèse de tels copolymères sont par exemple l'acide acrylique, l'acide méthacrylique, l'acide acrylamido-2-méthylpropanesulfonique, l'acide crotonique et l'acide itaconique, ou un mélange de ces acides.

Comme monomères non-ioniques, on peut citer par exemple les acrylates et méthacrylates d'alkyle en C₁₋₄, l'acrylamide, l'acétate de vinyle, le para-tertiobutylbenzoate de vinyle, le méthacrylamide, les N-mono-(alkyle en C₁₋₈)-acrylamides, le N-vinylformamide, le N-vinylacétonamide, l'acrylate d'hydroxypropyle et le méthacrylate d'hydroxypropyle.

Les monomères cationiques, utilisés en combinaison avec les monomères anioniques et non-ioniques pour la synthèse de copolymères amphotères utilisables dans la présente invention, sont choisis par exemple parmi les sels de di(alkyle en C₁₋₄)-diallylammonium et les composés de formule (I) 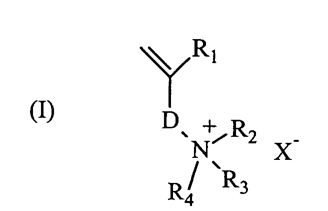 dans laquelle
R₁ représente un atome d'hydrogène ou un groupe méthyle,
R₂ et R₃, identiques ou différents, représentent chacun un atome d'hydrogène ou un groupe alkyle en C₁₋₄ linéaire ou ramifié,
R₄ représente un atome d'hydrogène, un groupe alkyle en C₁₋₄ linéaire ou ramifié ou un groupe aryle,
D représente le motif suivant 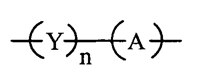 dans lequel Y représente une fonction amide, ester, uréthane ou urée,
A représente un groupe alkyène en C₁₋₁₀ linéaire, ramifié ou cyclique, pouvant être substitué ou interrompu par un cycle aromatique ou hétéroaromatique divalent, ou pouvant être interrompu par un hétéroatome choisi parmi O, N, S et P, et pouvant comporter une fonction cétone, amide, ester, uréthane ou urée,
n vaut 0 ou 1, et
X⁻ représente un contre-ion anionique tel qu'un ion halogénure ou sulfate.

On peut citer à titre d'exemples de copolymères préférés
- les copolymères d'acide (méth)acrylique et de (méth)acrylate d'alkyle en C₁₋₆ (nom INCI: Acrylates Copolymer), commercialisés par exemple sous la dénomination Amerhold® DR-25 par la société AMERCHOL,
- les copolymères d'acétate de vinyle, de para-tertiobutylbenzoate de vinyle et d'acide crotonique (nom INCI : VA/Vinyl Butyl Benzoate/Crotonates Copolymer), commercialisé par exemple sous la dénomination Mexomer® PW par la société CHIMEX, ou
- les copolymères d'octylacrylamide, de méthacrylate de tert-butylaminoéthyle et d'un ou de plusieurs monomères choisis parmi l'acide acrylique l'acide méthacrylique et les esters d'alkyle en C₁₋₆ de ces acides (nom INCI : Octylacrylamide/Acrylates/Butylaminoethyl Methacrylate Copolymer) commercialisés par exemple sous la dénomination Amphomer® par la société National Starch.

Les polymères anioniques ou amphotères utilisables dans la présente invention ne sont toutefois aucunement limités aux copolymères obtenus par polyaddition tels que décrits ci-dessus. On peut également envisager l'utilisation de polycondensats tels que des polyesters, polyamides ou polyuréthanes, ou de copolymères et dérivés de ceux-ci tels que les copolymères siliconés, portant un nombre suffisant de charges anioniques et éventuellement de charges cationiques pour être solubles dans le milieu liquide du composant (A) et suffisamment hydrophobes pour former un précipité solide en cas d'augmentation de la force ionique.

On peut citer à titre d'exemples de ce type de polymères
- le produit Luviset® Si-P.U.R.A (Nom INCI proposé : Polyurethane-6), commercialisé par la société BASF Aktiengesellschaft, qui est un polyuréthane siliconé neutralisé, en solution hydroalcoolique, résultant de la polycondensation d'acide isophtalique, d'acide adipique, d'hexylèneglycol, de néopentylglycol, d'acide diméthylolpropionique, d'isophorone-diisocyanate et de bis-éthylaminoisobutyldiméthicone,
- ou le produit commercialisé par la société AMERCHOL sous la dénomination Viscophobe® DB-1000 (nom INCI : Polyacrylate-3).

Le polymère anionique ou amphotère est de préférence présent dans la solution du composant A en une concentration suffisante pour permettre la formation d'un dépôt de polymère précipité capable de rigidifier les fibres kératiniques pendant le temps nécessaire à la destruction et reconstruction des ponts disulfures de la kératine. On obtient généralement une quantité et qualité de précipité satisfaisantes pour une concentration en polymère anionique ou amphotère au moins égale à 5 %, de préférence comprise entre 7 et 30 % en poids, par rapport au poids total de la solution appliquée dans l'étape (a).

Pour augmenter la force ionique du milieu de réduction jusqu'à une valeur suffisante, on peut utiliser en principe n'importe quel sel organique ou minéral qui n'interfère pas avec la réaction de réduction des ponts disulfure. On préfère en particulier les sels d'ammonium et les sels de métaux alcalins.

Lorsque les cations sont apportés par l'agent réducteur (sous forme de sel) celui-ci comporte une ou plusieurs fonctions carboxylate. On peut citer à titre d'exemple le thioglycolate d'ammonium ou le thiolactate d'ammonium.

Les cations métalliques ou ammonium peuvent également provenir d'hydroxydes minéraux. A titre d'exemples de sels minéraux utilisables, on peut citer les halogénures de métaux alcalins tels que le chlorure de sodium. Les sels organiques peuvent être par exemple le citrate d'ammonium, et les hydroxydes minéraux peuvent être la soude (NaOH) ou l'ammoniaque (NH₄OH).

L'étape de réduction du procédé de la présente invention est de préférence mise en oeuvre à un pH compris entre 7 et 11, en particulier entre 7,5 et 9,5.

L'homme du métier saura choisir, grâce à ses connaissances techniques générales, les agents d'ajustement du pH et agents tampon appropriés pour ajuster le pH des composants A et B à une valeur adaptée.

L'agent réducteur de la composition réductrice peut être n'importe quel agent réducteur connu, choisi parmi ceux utilisés habituellement dans le domaine de la déformation permanente des cheveux, tels que les sulfites, les bisulfites et les thiols. Parmi ces agents réducteurs, on préfère en particulier la cystéine, la cystéamine, l'acide thiolactique et l'acide thioglycolique et les sels cosmétiquement acceptables de ceux-ci, en particulier le thioglycolate d'ammonium.

La concentration de cet agent réducteur dans la composition réductrice est de préférence comprise entre 1 % et 25 % en poids, en particulier entre 1 et 10 % en poids.

Après application de la composition réductrice chargée en cations métalliques et/ou ammonium (sous forme de sels minéraux ou organiques) et précipitation du polymère, on donne aux cheveux la forme désirée en utilisant simplement les mains ou bien des moyens mécaniques simples permettant de maintenir facilement en place des mèches de cheveux plus longues, tels que des pinces, peignes, barrettes, boudins et élastiques. Cette mise en place peut être réalisée par l'utilisateur lui-même et ne nécessite pas l'intervention d'un coiffeur.

On laisse ensuite agir l'agent réducteur pendant un temps suffisant pour obtenir la réduction souhaitée. Ce temps de pose est de préférence compris entre 2 et 30 minutes, et en particulier entre 5 et 15 minutes.

Il est possible d'insérer entre l'étape de réduction (c) et l'étape d'oxydation (d) une étape de rinçage intermédiaire. Il convient toutefois de veiller à ce que ce rinçage laisse une quantité suffisante de précipité pour imposer aux cheveux une forme lors de l'étape d'oxydation.

Dans un mode de réalisation préféré de la présente invention, le procédé ne comporte pas d'étape de rinçage intermédiaire (entre l'étape de réduction (c) et l'étape d'oxydation (d)). Un tel rinçage pourrait en effet fragiliser ou éliminer le dépôt de polymère précipité, détruisant ainsi la mise en forme de la chevelure pendant l'étape de fixation.

La composition fixatrice est donc appliquée de préférence sur les cheveux directement après le premier temps de pose. Cette application se fait bien entendu dans la mesure du possible sans modification de la forme imposée à la chevelure dans l'étape précédente. Une telle application peut se faire par exemple à l'aide d'un dispositif aérosol dispensant une mousse fixatrice ou de fines gouttelettes d'une solution fixatrice. La nature de l'agent oxydant utilisé dans cette composition fixatrice n'est pas déterminante pour la présente invention et l'on peut utiliser n'importe quel agent oxydant connu utilisé habituellement pour l'oxydation des cheveux, tels que le peroxyde d'hydrogène, le peroxyde d'urée, les bromates, les persels et les mélanges de ceux-ci.

Le temps de pose pour l'étape de fixation est généralement compris entre 2 et 30 minutes, de préférence entre 2 et 15 minutes.

La composition restante sur les cheveux est ensuite éliminée par rinçage, suivi éventuellement d'un lavage et/ou d'un autre traitement capillaire.

Chacune des trois compositions de l'agent multicomposant peut contenir en outre des principes actifs et adjuvants cosmétiques utilisés couramment dans le domaine capillaire. Ces additifs sont choisis par exemple parmi les vitamines, les acides aminés, les oligopeptides, les peptides, les protéines hydrolysées ou non, modifiées ou non, les enzymes, les acides et alcools gras ramifiés ou non, les cires animales, végétales ou minérales, les céramides et les pseudo-céramides, les acides organiques hydroxylés, les filtres UV, les agents anti-oxydants et les agents anti-radicaux libres, les agents chélatants, les agents antipelliculaires, les agents régulateurs de séborrhée, les agents apaisants, les agents tensioactifs cationiques, anioniques, non-ioniques ou amphotères, les polymères cationiques, anioniques, neutres ou amphotères, les silicones organomodifiées ou non, les huiles minérales, végétales ou animales, les polyisobutènes et poly(α-oléfines), les esters gras, les agents colorants capillaires tels que les colorants directs et les pigments

L'exemple ci-après illustre un agent multicomposants pour la déformation des cheveux.

### Exemple

| Composant A | |
|---|---|
| Luviset Si-P.U.R.A | 66,6 g |
| Eau déminéralisée | q.s.p. 100 g |

| Composant B | |
|---|---|
| Acide thioglycolique | 5,1 g |
| Sel pentasodique de l'acide diéthylènetriaminopenta-acétique | 0,4 g |
| Ammoniaque | 5,6 g |
| Chlorure d'ammonium | 5,0 g |
| Hydroxypropylguar | 0,8 g |
| Mexomere® PO* (Chimex) | 1,6 g |
| Tégobétaine® HS** (Goldschmidt) | 1,4 g |
| Dithiodiglycolate d'ammonium | 1,6 g |
| Glycérol | 3,0 g |
| Parfum, peptisant | q.s. |
| Eau déminéralisée | q.s.p. 100 g |

| | |
|---|---|
| * polymère de N,N,N,N-tétraméthylhexaméthylènediamine et de chlorure de triméthylène (INCI : Hexadimethrine Chloride) | |
| ** cocamidopropylbétaïne et laurate de glycérol | |

| Composant C (composition fixatrice) | |
|---|---|
| Peroxyde d'hydrogène en solution à 50 % | 4,8 g |
| Stabilisants | 0,03 g |
| Laurylsulfate d'ammonium | 4,0 g |
| Acide citrique | q.s.p. pH 3 |
| Parfum, peptisant | q.s. |
| Eau déminéralisée | q.s.p. 100 g |

On a utilisé ces trois composants conformément au procédé de la présente invention pour une déformation permanente des cheveux. Sur l'ensemble des modèles, les cheveux traités ont de la texture et de la masse. Ils sont faciles à coiffer, modelables et volumineux.

Les effets cosmétiques observés sont conservés durablement pendant un grand nombre de shampooings.

Lorsqu'on utilise le procédé de la présente invention sur des cheveux ayant subi au préalable une coloration capillaire, celle-ci ne souffre pas du traitement de déformation permanente.

## Revendications

1. Procédé de déformation permanente des cheveux, comprenant, dans l'ordre, les étapes suivantes :
(a) application, sur les zones à déformer, d'une solution d'un polymère anionique ou amphotère dans un milieu contenant de l'eau et, éventuellement, un ou plusieurs solvants cosmétiquement acceptables,
(b) application, sur les zones traitées dans l'étape (a), d'une composition réductrice contenant un agent réducteur capable de couper les ponts disulfure de la kératine et une quantité suffisante de cations métalliques et/ou d'ions ammonium pour faire précipiter *in situ* le polymère anionique ou amphotère,
(c) mise en forme des cheveux ainsi traités suivie d'un temps de pose de la composition réductrice,
(d) application, sur les zones traitées dans les étapes (a) et (b), d'une composition fixatrice contenant un agent oxydant, suivie d'un temps de pose de la composition oxydante, et
(e) rinçage des cheveux.

2. Procédé selon la revendication 1, **caractérisé par le fait qu'**il comprend en outre, entre l'étape (c) et l'étape (d), une étape de rinçage intermédiaire laissant une quantité suffisante de précipité pour imposer aux cheveux une forme lors de l'étape d'oxydation (d).

3. Procédé selon la revendication 1 ou 2, **caractérisé par le fait que** le polymère anionique est un copolymère synthétique obtenu par polyaddition d'un mélange de monomères anioniques et de monomères non-ioniques.

4. Procédé selon la revendication 1 ou 2, **caractérisé par le fait que** le polymère amphotère est un copolymère synthétique obtenu par polyaddition d'un mélange de monomères anioniques, de monomères non-ioniques et de monomères cationiques.

5. Procédé selon la revendication 3 ou 4, **caractérisé par le fait que** les monomères anioniques sont choisis parmi l'acide acrylique, l'acide méthacrylique, l'acide acrylamido-2-méthylpropanesulfonique, l'acide crotonique et l'acide itaconique.

6. Procédé selon l'une quelconque des revendications 3 à 5, **caractérisé par le fait que** les monomères non-ioniques sont choisis parmi les acrylates et méthacrylates d'alkyle en C₁₋₄, l'acrylamide, l'acétate de vinyle, le para-tertiobutylbenzoate de vinyle, le méthacrylamide, les N-mono-(alkyle en C₁₋₈)-acrylamides, le N-vinylformamide, le N-vinylacétonamide, l'acrylate d'hydroxypropyle et le méthacrylate d'hydroxypropyle.

7. Procédé selon les revendications 4 à 6, **caractérisé par le fait que** les monomères cationiques sont choisis parmi les sels de di(alkyle en C₁₋₄)-diallylammonium et les composés de formule (I) 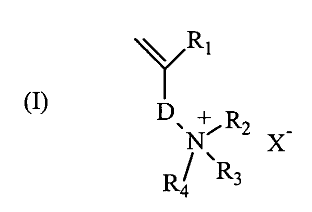 dans laquelle
R₁ représente un atome d'hydrogène ou un groupe méthyle,
R₂ et R₃, identiques ou différents, représentent chacun un atome d'hydrogène ou un groupe alkyle en C₁₋₄ linéaire ou ramifié,
R₄ représente un atome d'hydrogène, un groupe alkyle en C₁₋₄ linéaire ou ramifié ou un groupe aryle,
D représente le motif suivant 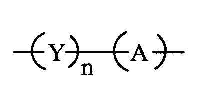 dans lequel
Y représente une fonction amide, ester, uréthane ou urée,
A représente un groupe alkyène en C₁₋₁₀ linéaire, ramifié ou cyclique, pouvant être substitué ou interrompu par un cycle aromatique ou hétéroaromatique divalent, ou pouvant être interrompu par un hétéroatome choisi parmi O, N, S et P, et pouvant comporter une fonction cétone, amide, ester, uréthane ou urée,
n vaut 0 ou 1, et
X⁻ représente un contre-ion anionique tel qu'un ion halogénure ou sulfate.

8. Procédé selon l'une quelconque des revendications précédentes, **caractérisé par le fait que** le polymère anionique ou amphotère est un copolymère d'acide (méth)acrylique et de (méth)acrylate d'alkyle en C₁₋₆, un copolymère acétate de vinyle/para-tertiobutylbenzoate de vinyle/acide crotonique ou un copolymère d'octylacrylamide de méthacrylate de tert-butylaminoéthyle et d'un ou de plusieurs monomères choisis parmi l'acide acrylique l'acide méthacrylique et les esters d'alkyle en C₁₋₆ de ces acides.

9. Procédé selon la revendication 1 ou 2, **caractérisé par le fait que** le polymère anionique ou amphotère est un polycondensat.

10. Procédé selon la revendication 9, **caractérisé par le fait que** le polymère anionique ou amphotère est un polyuréthanne, éventuellement siliconé.

11. Procédé selon l'une quelconque des revendications précédentes, **caractérisé par le fait que** la concentration du polymère anionique ou amphotère dans la solution appliquée dans l'étape (a) est au moins égale à 5 %, de préférence comprise entre 7 et 30 % en poids, par rapport au poids total de cette solution.

12. Procédé selon l'une quelconque des revendications précédentes, **caractérisé par le fait que** les cations métalliques et/ou ions ammonium sont introduit par ajout d'un sel minéral ou organique ou d'un hydroxyde, ou sont apportés par l'agent réducteur lorsqu'il est sous forme de sel.

13. Procédé selon l'une quelconque des revendications précédentes, **caractérisé par le fait que** la concentration des cations métalliques et/ou ammonium dans la composition réductrice est supérieure à 0,3 M, de préférence comprise entre 0,5 et 6 M.

14. Procédé selon l'une quelconque des revendications précédentes, **caractérisé par le fait que** l'agent réducteur est choisi parmi les sulfites, les bisulfites et les thiols.

15. Procédé selon la revendication 14, **caractérisé par le fait que** l'agent réducteur est choisi parmi la cystéine, la cystéarnine, l'acide thiolactique et l'acide thioglycolique et les sels cosmétiquement acceptables de ceux-ci, en particulier le thioglycolate d'ammonium.

16. Procédé selon l'une des revendications précédentes, **caractérisé par le fait que** la concentration de l'agent réducteur dans la composition réductrice est comprise entre 1 % et 25 % en poids, de préférence entre 1 et 10 % en poids.

17. Procédé selon l'une quelconque des revendications précédentes, **caractérisé par le fait que** le temps de pose de la solution réductrice est compris entre 2 et 30 minutes, de préférence entre 5 et 15 minutes.

18. Procédé selon l'une quelconque des revendications précédentes, **caractérisé par le fait que** l'agent oxydant dans la composition fixatrice est choisi parmi le peroxyde d'hydrogène, le peroxyde d'urée, les bromates, les persels et les mélanges de ceux-ci.

19. Procédé selon l'une quelconque des revendications précédentes, **caractérisé par le fait que** le temps de pose de la composition fixatrice est compris entre 2 et 30 minutes, de préférence entre 2 et 15 minutes.

20. Agent multicomposants pour la déformation permanente des cheveux comprenant,
• en tant que premier composant (composant A), une solution d'un polymère anionique ou amphotère dans un milieu contenant de l'eau et, éventuellement, un ou plusieurs solvants cosmétiquement acceptables, capable de former un précipité solide en présence d'une quantité suffisante de cations métalliques ou d'ions d'ammonium,
• en tant que deuxième composant (composant B), une composition réductrice contenant un agent réducteur capable de couper les ponts disulfure de la kératine, et une quantité suffisante de cations métalliques et/ou d'ions ammonium pour faire précipiter le polymère anionique ou amphotère du premier composant, et
• en tant que troisième composant (composant C), une composition oxydante contenant un agent oxydant capable de rétablir les ponts disulfure de la kératine

21. Agent multi-composants selon la revendication 18, **caractérisé par le fait qu'**il se présente sous forme d'un kit multi-compartiments comportant au moins trois compartiments contenant respectivement les trois composants A, B et C.
